# EUROPEAN PATENT SPECIFICATION

(11) **EP 3 115 076 B1**
(45) Date of publication and mention of the grant of the patent: **29.03.2023**
(21) Application number: 15758537.3
(22) Date of filing: 19.02.2015
(51) Int. Cl.: A61M 25/04, A61M 25/00

(54) **CATHETER**
KATHETER
CATHÉTER

(30) Priority: 04.03.2014 JP 2014041413
(43) Date of publication of application: 11.01.2017
(73) Proprietor: Terumo Kabushiki Kaisha, Tokyo 151-0072 (JP)
(72) Inventor: YAZAKI, Yushin, Fujinomiya-shi Shizuoka 418-0015 (JP)
(74) Representative: Casalonga
(86) International application number: PCT/JP2015/054514
(87) International publication number: WO 2015/133281

(56) References cited:
- EP-A2- 1 712 248
- WO-A2-03/026535
- JP-A- H0 871 161
- US-A- 5 224 939
- US-A1- 2012 071 833
- US-B1- 6 273 404

## Description

### Technical Field

The present invention relates to a catheter having an improved kink resistance.
In particular, the present invention relates to a catheter according to the preamble of claim 1, such as it is known for example from Background Art US 6 273 404 B1.

There are various types of catheters to be inserted into a living body depending on purposes. For example, an introducer sheath and a guiding sheath used to percutaneously introduce a treatment catheter, or the like into a living body represent a blood vessel indwelling catheter. In general, such a sheath includes a flexible tube-shaped main sheath body (main catheter body), a hub connected to a proximal portion of the main sheath body, and a strain relief that surrounds a predetermined range of a proximal side of the main sheath body to suppress the occurrence of a kink (for example, see JP-A-8-71161). Note that the strain relief is provided to another type of catheter to suppress the occurrence of a kink at a proximal side of a main catheter body.

Incidentally, a catheter requires compatibility between reducing a diameter to achieve a low invasion and ensuring an inner diameter to achieve a technique, and reducing a thickness of a main catheter body is a solution to the requirement. However, when only the thickness of the main catheter body is merely reduced, the possibility that a kink will occur at the time of operating the catheter increases.

### Disclosure of the Invention

The invention has been conceived in view of the above problem, and an object of the invention is to provide a catheter capable of increasing kink resistance of a main catheter body while a thickness is reduced.

To achieve the above object, the invention provides a catheter according to claim 1. The dependent claims relate to advantageous embodiments. 2

According to the catheter of the invention configured as described above, when the catheter is treated in a direction in which the hard portion is at a side with respect to a direction in which the main catheter body is bent at the time of use, a proximal-side portion of the main catheter body surrounded by the strain relief is supported by the hard portion when the main catheter body is bent. The proximal-side portion of the main catheter body supported by the hard portion, rigidity of which is higher than that of the other portion, is inhibited from being deformed in an outside direction (horizontal direction), and thus stress concentration corresponding to a source of a kink is relieved. In this way, the occurrence of a kink in the proximal-side portion of the main catheter body may be effectively prevented or suppressed. Therefore, kink resistance of the main catheter body may be increased while decreasing a thickness.

In the above-described catheter, a side port may be provided in an outer circumferential portion of the hub, and a circumferential direction position of one hard portion in the strain relief may correspond to a circumferential direction position of the hub in which the side port is provided. When the catheter is indwelled, the side port is normally laterally arranged. Thus, in normal use, the hard portion is inevitably positioned at a side with respect to a bending direction of the proximal-side portion of the main catheter body. Therefore, kink resistance performance may be effectively exhibited without a user paying attention to a direction of the hard portion. In addition, a kink of the main catheter body may be prevented by the hard portion of the strain relief without adjusting a position of the strain relief. Thus, operation man-hours may be reduced, and a burden on an operator may be lightened.

In the above-described catheter, the hard portion contains a harder material than a material of the other portion. In this way, the hard portion may be easily configured.

In the above-described catheter, the hard portion may be configured by adding a reinforcement body to the strain relief. In this way, the hard portion may be easily configured.

In the above-described catheter, the catheter may be an introducer sheath indwelled in a lumen of a living body to introduce another long medical instrument into the body. In this way, it is possible to provide an introducer sheath excellent in kink resistance.

According to the catheter of the invention, kink resistance of the main catheter body may be increased while reducing a thickness.

### Brief Description of the Drawings

Fig. 1 is a partially-omitted schematic cross-sectional view of a catheter according to a first embodiment of the invention.
Fig. 2A is a longitudinal sectional view taken along IIA-IIA line of Fig. 1, and Fig. 2B is a longitudinal sectional view in a state in which a proximal-side portion of a main catheter body is bent.
Fig. 3A is a cross-sectional view taken along IIIA-IIIA line of Fig. 2A, and Fig. 3B is a cross-sectional view taken along IIIB-IIIB line of Fig. 2B.
Fig. 4 is a diagram for description of a usage state of the catheter illustrated in Fig. 1.
Fig. 5A is a cross-sectional view of the main catheter body and a strain relief according to a first example, and Fig. 5B is a cross-sectional view of the main catheter body and a strain relief according to a second example. Best Mode for Carrying Out the Invention

Hereinafter, a catheter according to the invention will be described using a preferred embodiment with reference to accompanying drawings.

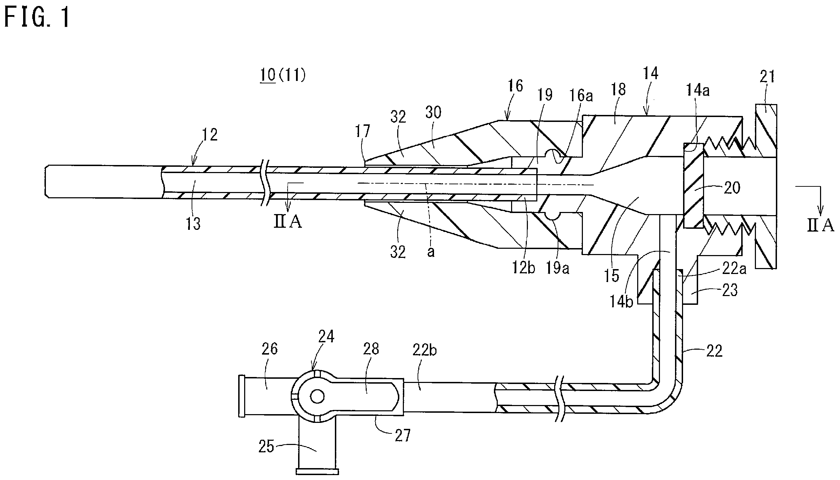

Fig. 1 is a partially-omitted schematic cross-sectional view of a catheter 10 according to a first embodiment of the invention. The catheter 10 is a medical instrument inserted into a lumen in a living body such as a blood vessel, and is configured as an introducer sheath 11 in the present embodiment. The introducer sheath 11 is a device used in combination with a dilator (not illustrated) to introduce a treatment catheter, or the like percutaneously into the living body. Note that the catheter 10 may be configured as a catheter other than the introducer sheath 11, for example, a guiding sheath, a treatment catheter such as a balloon catheter, a guiding catheter, or the like.

As illustrated in Fig. 1, the catheter 10 includes a main catheter body 12, a hub 14, and a strain relief 16. The main catheter body 12 is a flexible tube-shaped member, and is also referred to as a shaft. In the case of the introducer sheath 11, the main catheter body 12 is also referred to as a sheath tube. The main catheter body 12 has a lumen 13 opened at a distal end and a proximal end of the main catheter body 12.

A length of the main catheter body 12 varies according to type (intended use) of the catheter 10. For example, in the case of the introducer sheath 11, the length is in a range of about 30 to 800 mm.

Examples of a material included in the main catheter body 12 include a polyvinyl chloride-based resin, a urethane resin such as polyurethane, an olefin-based resin such as polyethylene, polyamide, a synthetic resin such as ethylene-vinyl acetate copolymer, silicone rubber, latex rubber, fluorocarbon resin, aromatic polyether ketone, and stainless steel.

The hub 14 is a member that is connected to a proximal portion 12b of the main catheter body 12 and includes a lumen 15 to communicate with the lumen 13 of the main catheter body 12. A protruding portion 19, a diameter of which is reduced with respect to a body portion 18 of the hub 14, is provided to protrude in a distal end direction at a distal end of the hub 14, and the proximal portion 12b of the main catheter body 12 is inserted into and fixed to an inner peripheral portion of a distal end of the protruding portion 19. Examples of a constituent material of the hub 14 include a rigid resin such as polypropylene, polyethylene, ABS, and polycarbonate.

A valve body 20 for preventing a leakage of a liquid from the inside of the hub 14 is disposed inside the hub 14. A hollow fixing member 21 is screwed with a proximal portion of the hub 14, and the valve body 20 is fixed inside the hub 14 when the valve body 20 is interposed between a distal surface of the fixing member 21 and a step portion 14a formed inside the hub 14. Note that a scheme of fixing the fixing member 21 to the proximal portion of the hub 14 is not restricted to screwing, and a structure capable of interposing the valve body 20 between the hub 14 and the fixing member 21 may be used. For example, the fixing member 21 may be bonded or heat-welded to the hub 14 such that the valve body 20 is interposed between the hub 14 and the fixing member 21. In addition, instead of not providing the valve body 20 in the hub 14, the valve body 20 may be provided in a connector member connectable to a proximal end of the hub 14.

The valve body 20 has an elastic material (for example, silicone rubber). A slit (not illustrated) into which a dilator or another catheter can be inserted is formed in the valve body 20. The valve body 20 may prevent a liquid (blood, or the like) flowing in the lumen 15 of the hub 14 through the main catheter body 12 from leaking out in a state in which the dilator, or the like is inserted into the slit.

A flexible side tube 22 is connected to a side portion of the hub 14. In an illustrated example, one end of the side tube 22 is connected to a side port 23 extruding outward from the side portion of the hub 14. A lumen of the side tube 22 communicates with the lumen 15 of the hub 14 through a side hole 14b provided in the hub 14.

A three-way stopcock 24 is provided at an end portion 22b of the side tube 22 on the opposite side from an end portion 22a connected to the hub 14. For example, the three-way stopcock 24 includes a port 25 for discharging air, a port 26 for chemical injection to which a syringe (not illustrated) is connected, a port 27 connected to the end portion 22b of the side tube 22, and a cock 28 for switching communication states of the ports 25, 26, and 27. Note that the port 25 and the port 26 are not restricted to discharging air and chemical injection, and use is not particularly restricted.

The strain relief 16 has a function of preventing or suppressing a kink (bending) at a proximal side of the main catheter body 12 (specifically, an interlock portion of the main catheter body 12 and the hub 14 and a region around the interlock portion). As illustrated in Fig. 1, the strain relief 16 is a hollow member having flexibility which is supported at a distal portion of the hub 14 (protruding portion 19) and surrounds a predetermined range of the proximal side of the main catheter body 12.

An outer diameter of the strain relief 16 is relatively large at a proximal side thereof and relatively small at a distal side thereof, and the outer diameter gradually decreases toward the distal side. A wall thickness of the strain relief 16 decreases toward the distal side. A proximal portion of the strain relief 16 is fit onto the protruding portion 19 provided at the distal end of the hub 14. In this way, the strain relief 16 is supported by the distal portion of the hub 14.

In Fig. 1, an inner diameter of a distal-side portion of the strain relief 16 is set to be slightly larger than an outer diameter of the main catheter body 12 to facilitate assembly at the time of manufacturing the catheter 10. However, the inner diameter of the distal-side portion of the strain relief 16 may be nearly the same as the outer diameter of the main catheter body 12. In a case in which a proximal-side portion of the main catheter body 12 is bent, a kink is prevented when the distal-side portion of the strain relief 16 supports the proximal-side portion of the main catheter body 12. Hereinafter, a region of the strain relief 16 which supports the proximal-side portion in a case in which the proximal-side portion of the main catheter body 12 is bent (the distal-side portion of the strain relief 16) is referred to as a "support portion 30".

Note that a bulge portion 19a annularly extending in a circumferential direction is formed on an outer peripheral surface of the protruding portion 19 in Fig. 1, and the strain relief 16 is prevented from falling out of the protruding portion 19 when the bulge portion 19a is engaged with an annular depression 16a formed on an inner peripheral surface of the proximal portion of the strain relief 16.

Examples of a constituent material of the strain relief 16 include a thermoplastic resin such as styrene resin, olefin resin, and polyester resin, or an elastic material such as silicone rubber.

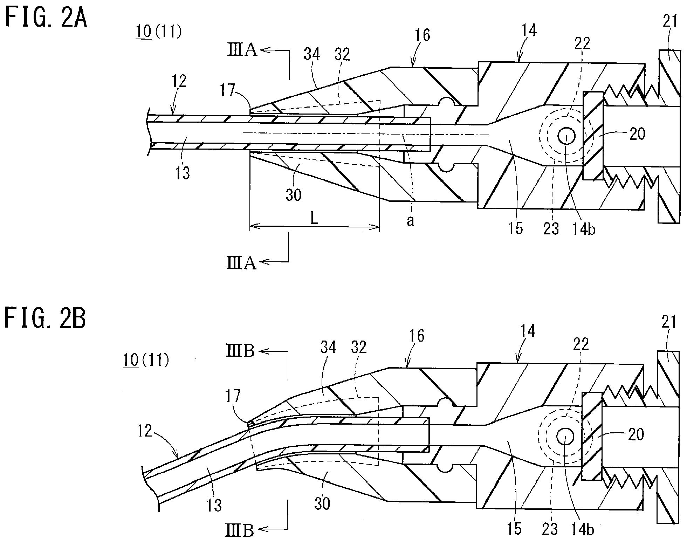

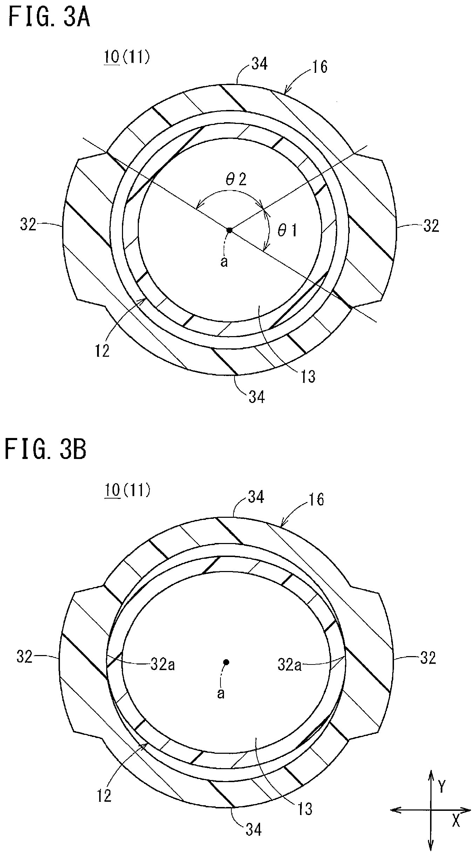

Fig. 2A is a longitudinal sectional view taken along IIA-IIA line of Fig. 1, and Fig. 2B is a longitudinal sectional view in a state in which the proximal-side portion of the main catheter body 12 is bent. Fig. 3A is a cross-sectional view taken along IIIA-IIIA line of Fig. 2A. As illustrated in Fig. 2A and Fig. 3A, the strain relief 16 has a hard portion 32 at least in the distal-side portion (support portion 30). The hard portion 32 is provided in each of regions opposite from each other with respect to an axis a of the strain relief 16, and is configured to be harder than another portion 34 in a circumferential direction at the same axial direction position.

The other portion 34 in the strain relief 16 is a portion which is present at a circumferential direction position between one and the other hard portions 32, and is present in each of regions opposite from each other with respect to the axis a of the strain relief 16.

A circumferential direction position of the one hard portion 32 in the strain relief 16 corresponds to a circumferential direction position of the hub 14 at which the side port 23 is provided. A circumferential direction position of the other hard portion 32 in the strain relief 16 corresponds to a circumferential direction position on the opposite side from the circumferential direction position of the hub 14 at which the side port 23 is provided.

The hard portion 32 is provided in a predetermined section from a most distal portion 17 of the strain relief 16. For example, a length L of the hard portion 32 along an extending direction of the main catheter body 12 is 3 to 10 mm, and preferably set to 4 to 7 mm. The hard portion 32 may be provided across a whole length of the support portion 30, or may be provided in a portion of the whole length of the support portion 30 (however, the most distal portion 17 of the support portion 30 should be included) . The hard portion 32 may be provided continuously or discontinuously along the extending direction of the main catheter body 12.

The catheter 10 according to the present embodiment basically configured as described above. Hereinafter, operations and effects thereof will be described.

As described in the foregoing, the catheter 10 is configured as the introducer sheath 11. In the use of the introducer sheath 11, the introducer sheath 11 is combined with a dilator (not illustrated). Specifically, an assembly (introducer) in a state in which the dilator is inserted into the introducer sheath 11 punctures a blood vessel of a patient such that a distal end of the introducer sheath 11 secures the blood vessel. Thereafter, the main catheter body 12 is further inserted into the blood vessel and inserted up to a portion around the most distal portion 17 of the support portion 30, and then the dilator is pulled out of the introducer sheath 11. A length in which the main catheter body 12 is inserted into the blood vessel is set to a predetermined length.

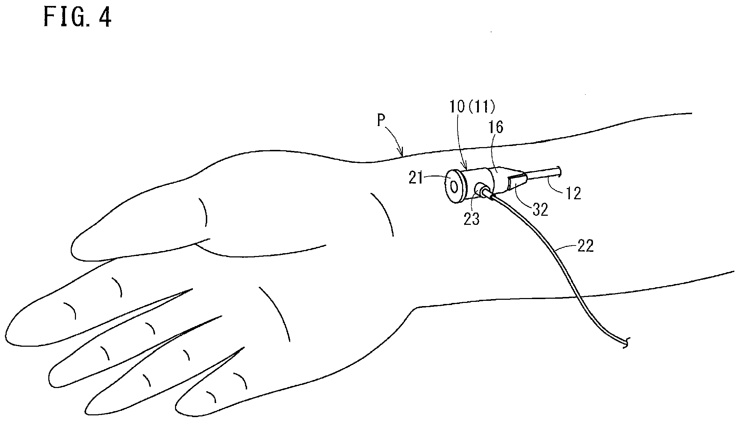

Fig. 4 is a diagram for description of a state in which the introducer sheath 11 is indwelled in a blood vessel of a patient P. As in Fig. 4, the introducer sheath 11 is indwelled by disposing the side port 23 provided in the hub 14 laterally (in a direction along a body surface of the patient P). This is because the side port 23 and the side tube 22 interfere with a subsequent procedure when the introducer sheath 11 is indwelled while the side port 23 is pointed upward in Fig. 4.

As described above, a direction of the side port 23 at the time of indwelling the introducer sheath 11 is practically determined. Therefore, a direction in which the proximal-side portion of the main catheter body 12 is bent at the time of inserting and removing another device (a treatment catheter, or the like) into and from the introducer sheath 11 is determined. In the case of Fig. 4, a direction perpendicular to the direction of the side port 23 (a direction perpendicular to a surface of a body) is the direction in which the proximal-side portion of the main catheter body 12 is bent.

After indwelling the introducer sheath 11 as described above, another device such as a treatment catheter (balloon catheter, or the like) or a guiding catheter is introduced through the introducer sheath 11 while being led by a guide wire (not illustrated) inside the blood vessel. Then, the device is advanced up to a predetermined region inside the living body, and angiography or treatment is performed.

Incidentally, since the proximal-side portion of the main catheter body 12 (the interlock portion of the main catheter body 12 and the hub 14) is exposed to the outside of the body during a surgery, the hub 14 may be lifted to insert and remove another device from a proximal end opening of the hub 14. In this case, the proximal-side portion of the main catheter body 12 is bent as in Fig. 2B, and bending stress acts on the proximal-side portion. The strain relief 16 is provided to inhibit a kink from being generated in the proximal-side portion of the main catheter body 12 due to the bending stress at this time.

A conventional strain relief exhibits kink resistance to a certain extent. However, stress may be concentrated on a proximal-side portion of a main catheter body to cause a kink. On the other hand, in the case of the strain relief 16 of the catheter 10 according to the present embodiment, since the hard portion 32 configured to be harder than the other portion 34 is provided, the occurrence of a kink may be suitably prevented or suppressed by preventing stress from being concentrated on the proximal-side portion of the main catheter body 12.

Specifically, when the proximal-side portion of the main catheter body 12 is bent, deformation of the proximal-side portion of the main catheter body 12 in a horizontal direction (X direction) is regulated (suppressed) by being supported by an inner surface 32a of the hard portion 32 of the strain relief 16 from both sides as in Fig. 3B which is a cross-sectional view along IIIB-IIIB line of Fig. 2B. That is, when the proximal-side portion of the main catheter body 12 is bent as in Fig. 2B, the proximal-side portion is crushed in a Y direction of Fig. 3B and is about to be extended in the X direction (is about to be in an oval shape) . However, the hard portion 32, rigidity of which is higher than the other portion 34, suppresses deformation of the main catheter body 12 in the X direction. As a result, crushing of the proximal-side portion of the main catheter body 12 in the Y-direction is suppressed, and stress concentration corresponding to a source of a kink is relieved.

In addition, unlike the hard portion 32, the other portion 34, which is bent together with the proximal-side portion of the main catheter body 12 in response to bending of the proximal-side portion, has moderate flexibility. For this reason, flexibility of the strain relief 16 in a bending direction is properly ensured, and it is possible to prevent stress from being concentrated on one point of a contact portion of the proximal-side portion of the main catheter body 12 and the strain relief 16 to cause a source of a kink. Specifically, when a portion of the strain relief 16 other than the hard portion 32 retains flexibility while the main catheter body 12 is bent, a contact area of the main catheter body 12 and the portion of the proximal-side portion of the strain relief 16 other than the hard portion 32 is widened at a center side (inner side) of bending of the main catheter body 12. For this reason, the strain relief 16 and the proximal-side portion of the main catheter body 12 may be inhibited from coming into contact with each other at one point.

Incidentally, as an angle range **θ**1 in which the hard portion 32 is provided increases, an angle range **θ**2 of the other portion 34 decreases (see Fig. 3A). As described above, the hard portion 32 supports the main catheter body 12 from the both sides when the main catheter body 12 is bent, thereby preventing a source of a kink from being caused. Meanwhile, since the other portion 34 is a portion which is bent together with the main catheter body 12 in response to bending of the main catheter body 12, moderate flexibility is preferably ensured. However, when the angle range **θ**2 of the other portion 34 becomes excessively smaller, there is difficulty in ensuring flexibility of the bending direction.

Accordingly, for example, the angle range **θ**1 in which the hard portion 32 is provided is preferably set to 10° < **θ**1 ≤ 120°, and more preferably set to 40° ≤ **θ**1 ≤ 80°. In this way, the angle range **θ**2 of the other portion 34 is greater than or equal to 60°, and moderate flexibility is ensured. Thus, it is possible to prevent stress from being concentrated on one point of the main catheter body 12 at the time of bending, and a source of a kink from being caused.

As described in the foregoing, according to the catheter 10, when the main catheter body 12 is bent, the proximal-side portion of the main catheter body 12 surrounded by the strain relief 16 is supported by the hard portion 32 from the both sides . The main catheter body 12 supported by the hard portion 32, rigidity of which is higher than that of the other portion 34, is inhibited from being deformed in the horizontal direction. Thus, stress concentration corresponding to a source of a kink is relieved, and the occurrence of a kink in the proximal-side portion of the main catheter body 12 may be effectively prevented or suppressed. Therefore, kink resistance of the main catheter body 12 may be increased while a thickness is reduced.

As described in the foregoing, when the introducer sheath 11 is indwelled inside the blood vessel, the main catheter body 12 is inserted into the blood vessel up to a portion around the most distal portion 17 of the support portion 30. For this reason, in particular, a portion around the support portion 30 of the main catheter body 12 fixedly comes into contact with a skin or a blood vessel wall, and a bending degree locally increases easily in the portion. Thus, a kink easily occurs. For this reason, the invention capable of suppressing a kink in the proximal-side portion of the main catheter body 12 is suitable for the introducer sheath 11.

In addition, when the introducer sheath 11 is indwelled inside the blood vessel as in Fig. 4, the introducer sheath 11 and the body may be loosely fixed using an adhesive tape by spreading the adhesive tape from above the strain relief 16 (not a portion of the hub 14) such that the main catheter body 12 does not rotate. In this case, since the hub 14 is merely loosely fixed by the adhesive tape, the hub 14 may be lifted (the main catheter body 12 may be bent). However, a force is easily applied to a portion around the support portion 30. For this reason, the invention capable of suppressing a kink in the proximal-side portion of the main catheter body 12 is useful.

As described in the foregoing, the circumferential direction position of the one hard portion 32 in the strain relief 16 corresponds to the circumferential direction position of the hub 14 at which the side port 23 is provided. The circumferential direction position of the other hard portion 32 in the strain relief 16 corresponds to the circumferential direction position on the opposite side from the circumferential direction position of the hub 14 at which the side port 23 is provided. When the catheter 10 is indwelled, the side port 23 is normally laterally arranged. Thus, in normal use, the hard portion 32 is inevitably positioned at a side with respect to the bending direction of the proximal-side portion of the main catheter body 12 (see Fig. 4). Therefore, kink resistance performance may be effectively exhibited without a user paying attention to a direction of the hard portion 32. In addition, a kink of the main catheter body 12 may be prevented by the hard portion 32 of the strain relief 16 without adjusting a position of the strain relief 16. Thus, operation man-hours may be reduced, and a burden on an operator may be lightened.

The side port 23 may not be provided in the hub 14. Even in this case, when the catheter 10 is treated in a direction in which the hard portion 32 is at a side with respect to a direction in which the main catheter body 12 is bent at the time of use, the proximal-side portion of the main catheter body 12 surrounded by the strain relief 16 is supported by the hard portion 32 when the main catheter body 12 is bent. Therefore, the occurrence of a kink in the proximal-side portion of the main catheter body 12 may be effectively prevented or suppressed.

In addition, when a certain mark, or the like is provided on an outer peripheral surface of the hard portion 32 such that a position of the hard portion 32 can be recognized from appearance, the catheter 10 may be easily treated in the direction in which the hard portion 32 is at the side with respect to the direction in which the main catheter body 12 is bent at the time of use.

The strain relief 16 may be rotatable with respect to the main catheter body 12. In this case, when the mark, or the like is provided in the hard portion 32, a position of the hard portion 32 may be easily adjusted after indwelling the catheter 10. That is, positioning may be easily performed such that the hard portion 32 is positioned at a side with respect to the bending direction of the proximal-side portion of the main catheter body 12 by rotating the strain relief 16 with respect to the main catheter body 12.

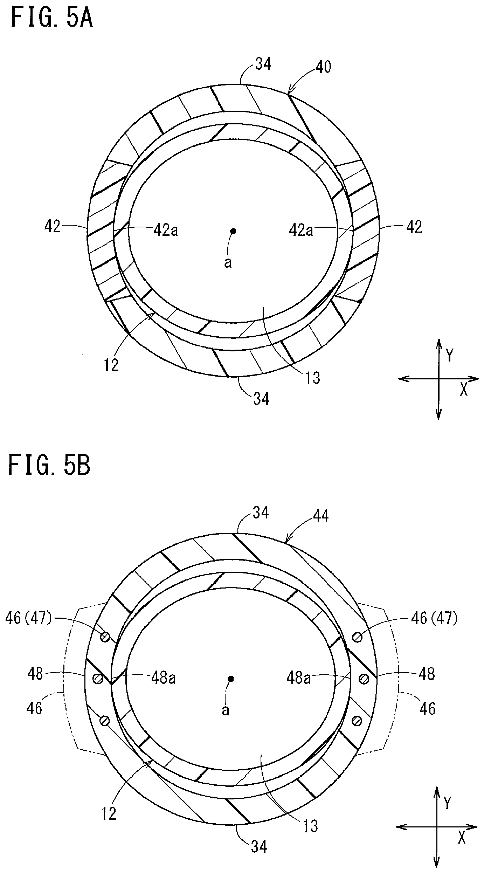

A hard portion 42 containing a harder material than that of another portion 34 is provided in each of regions opposite from each other with reference to an axis a as in a strain relief 40 illustrated in Fig. 5A. Examples of a material contained in the hard portion 42 include a hard olefin resin such as high molecular weight polyethylene or polypropylene, a resin such as polyimide, polyethylene terephthalate, rigid polyvinyl chloride, acrylic resin, polycarbonate, acrylonitrile-butadiene-styrene resin (ABS resin), polystyrene, or aromatic polyether ketone, and metal such as stainless steel. The hard portion 42 has the same wall thickness as that of the other portion 34 in the strain relief 40. However, the hard portion 42 is configured to be harder than the other portion 34 by containing a harder material than that of the other portion 34. That is, the hard portion 42 has higher rigidity than that of the other portion 34. Note that Fig. 5A illustrates a main catheter body 12 at the time of bending.

According to a configuration of Fig. 5A, a proximal-side portion of the main catheter body 12 surrounded by the strain relief 40 is supported by an inner surface 42a of the hard portion 42 from both sides when the main catheter body 12 is bent. Since the proximal-side portion supported by the hard portion 42, which has higher rigidity than that of the other portion 34, is inhibited from being deformed in a horizontal direction (X direction), stress concentration corresponding to a source of a kink is relieved. Therefore, kink resistance of the main catheter body 12 may be increased.

A hard portion 48 configured by adding a reinforcement body 46 thereto may be provided in each of regions opposite from each other with respect to an axis a as in a strain relief 44 according to a second example illustrated in Fig. 5B. The hard portion 48 has the same wall thickness as that of another portion 34. However, the hard portion 48 is configured to be harder than the other portion 34 by the added reinforcement body 46. That is, the hard portion 48 has higher rigidity than that of the other portion 34. Note that Fig. 5B illustrates a main catheter body 12 at the time of bending.

The reinforcement body 46 contains a harder material than a base material of the strain relief 44. Examples of the material contained in the reinforcement body 46 include a hard olefin resin such as high molecular weight polyethylene or polypropylene, a resin such as polyimide, polyethylene terephthalate, rigid polyvinyl chloride, acrylic resin, polycarbonate, acrylonitrile-butadiene-styrene resin (ABS resin), polystyrene, or aromatic polyether ketone, and metal such as stainless steel. For example, as illustrated in Fig. 5B, the reinforcement body 46 may be configured as a mesh member 47 (braid) formed by weaving a plurality of fine wires. Alternatively, the reinforcement body 46 may be a wire-shaped or bar-shaped member that extends along an extending direction of the main catheter body 12. The reinforcement body 46 may be fixed to an outer peripheral surface of the strain relief 44 as indicated by a virtual line of Fig. 5B instead of being embedded in a wall portion included in the strain relief 44.

According to a configuration of Fig. 5B, a proximal-side portion of the main catheter body 12 surrounded by the strain relief 44 is supported by an inner surface 48a of the hard portion 48 from both sides when the main catheter body 12 is bent. Since the proximal-side portion of the main catheter body 12 supported by the hard portion 48, which has higher rigidity than that of the other portion 34, is inhibited from being deformed in a horizontal direction (X direction), stress concentration corresponding to a source of a kink is relieved. Therefore, kink resistance of the main catheter body 12 may be increased.

Even though the invention has been described above using the preferred embodiment, the invention is not restricted to the embodiment, and may be variously modified within a range not departing from a subject matter of the invention.

## Claims

1. A catheter (10) comprising:
a flexible tube-shaped main catheter body (12);
a hub (14) connected to a proximal portion (12b) of the main catheter body (12); and
a flexible strain relief (16, 40, or 44) supported by a distal portion of the hub (14), the strain relief (16, 40, or 44) surrounding a predetermined range of a proximal side of the main catheter body (12), wherein the strain relief (16, 40, or 44) includes a hard portion (32, 42, or 48) which is provided in each of regions opposite from each other with respect to an axis in at least a distal-side portion, and which is configured to be harder than another portion (34) in a circumferential direction,
**characterized in that**
the hard portion (42) contains a harder material than a material of the other portion.

2. The catheter (10) according to claim 1,
**characterized in that** a side port (23) is provided in an outer circumferential portion of the hub (14), and
a circumferential direction position of one hard portion (32, 42, or 48) in the strain relief (16, 40, or 44) corresponds to a circumferential direction position of the hub (14) in which the side port (23) is provided.

3. The catheter (10) according to claim 1, **characterized in that** the hard portion (48) is configured by adding a reinforcement body (46) to the strain relief (44).

4. The catheter (10) according to any one of claims 1 to 2, **characterized in that** the catheter (10) is an introducer sheath (11) indwelled in a lumen of a living body to introduce another long medical instrument into the body.

## Patentansprüche

1. Katheter (10), umfassend:
einen flexiblen schlauchförmigen Hauptkatheterkörper (12) ;
eine Nabe (14), die mit einem proximalen Abschnitt (12b) des Hauptkatheterkörpers (12) verbunden ist; und
eine flexible Zugentlastung (16, 40 oder 44), die durch einen distalen Abschnitt der Nabe (14) gestützt wird, wobei die Zugentlastung (16, 40 oder 44) einen vorbestimmten Bereich einer proximalen Seite des Hauptkatheterkörpers (12) umgibt, wobei die Zugentlastung (16, 40 oder 44) einen harten Abschnitt (32, 42 oder 48) umfasst, der in jedem der Bereiche vorgesehen ist, die einander in Bezug auf eine Achse in mindestens einem distalseitigen Abschnitt gegenüberliegen, und der so konfiguriert ist, dass er härter ist als ein anderer Abschnitt (34) in einer Umfangsrichtung,
**dadurch gekennzeichnet, dass**
der harte Abschnitt (42) ein härteres Material enthält als ein Material des anderen Abschnitts.

2. Katheter (10) nach Anspruch 1,
**dadurch gekennzeichnet, dass** ein Seitenanschluss (23) in einem äußeren Umfangsabschnitt der Nabe (14) vorgesehen ist, und
eine Position in Umfangsrichtung eines harten Abschnitts (32, 42 oder 48) in der Zugentlastung (16, 40 oder 44) einer Position in Umfangsrichtung der Nabe (14) entspricht, in welcher der Seitenanschluss (23) vorgesehen ist.

3. Katheter (10) nach Anspruch 1, **dadurch gekennzeichnet, dass** der harte Abschnitt (48) durch Hinzufügen eines Verstärkungskörpers (46) zu der Zugentlastung (44) konfiguriert ist.

4. Katheter (10) nach einem der Ansprüche 1 bis 2, **dadurch gekennzeichnet, dass** der Katheter (10) eine Einführschleuse (11) ist, die in ein Lumen eines lebenden Körpers eingeführt ist, um ein anderes langes medizinisches Instrument in den Körper einzuführen.

## Revendications

1. Cathéter (10) comprenant :
un corps de cathéter principal (12) en forme de tube flexible ;
un moyeu (14) relié à une partie proximale (12b) du corps de cathéter principal (12) ; et
un réducteur de tension (16, 40, ou 44) flexible supporté par une partie distale du moyeu (14), le réducteur de tension (16, 40, ou 44) entourant une plage prédéterminée d'un côté proximal du corps de cathéter principal (12), dans lequel le réducteur de tension (16, 40, ou 44) comporte une partie dure (32, 42, ou 48) qui est prévue dans chacune des régions opposées les unes aux autres par rapport à un axe dans au moins une partie côté distal, et qui est configurée pour être plus dure qu'une autre partie (34) dans une direction circonférentielle,
**caractérisé en ce que**
la partie dure (42) contient un matériau plus dur qu'un matériau de l'autre partie.

2. Cathéter (10) selon la revendication 1,
**caractérisé en ce qu'**un orifice latéral (23) est prévu dans une partie circonférentielle extérieure du moyeu (14), et
une position de direction circonférentielle d'une partie dure (32, 42, ou 48) dans le réducteur de tension (16, 40, ou 44) correspond à une position de direction circonférentielle du moyeu (14) dans laquelle l'orifice latéral (23) est prévu.

3. Cathéter (10) selon la revendication 1, **caractérisé en ce que** la partie dure (48) est configurée par ajout d'un corps de renforcement (46) au réducteur de tension (44).

4. Cathéter (10) selon l'une quelconque des revendications 1 à 2, **caractérisé en ce que** le cathéter (10) est une gaine d'introduction (11) à demeure dans une lumière d'un corps vivant pour introduire un autre instrument médical long dans le corps.
